# EUROPEAN PATENT SPECIFICATION

(11) **EP 3 400 332 B1**
(45) Date of publication and mention of the grant of the patent: **23.12.2020**
(21) Application number: 17736319.9
(22) Date of filing: 05.01.2017
(51) Int. Cl.: D06P 1/94, D06P 3/79, D06P 1/20, D06P 1/24, A61B 17/04

(54) **METHODS FOR DYEING ULTRA-HIGH MOLECULAR WEIGHT POLYETHYLENE AND DYED ARTICLES MADE BY THE SAME**
VERFAHREN ZUR FÄRBUNG VON POLYETHYLEN MIT ULTRAHOHEM MOLEKULARGEWICHT UND DADURCH HERGESTELLTE GEFÄRBTE ARTIKEL
PROCÉDÉS PERMETTANT DE TEINDRE DU POLYÉTHYLÈNE À MASSE MOLÉCULAIRE ULTRA ÉLEVÉE ET ARTICLES TEINTS FABRIQUÉS À L'AIDE DE CEUX-CI

(30) Priority: 06.01.2016 US 201662275268 P
(43) Date of publication of application: 14.11.2018
(73) Proprietor: US Biodesign, Inc., Quakertown, PA 18951 (US)
(72) Inventor: FOOTE, Bruce, Lehighton PA 18235 (US); MOLZ, Thomas, R., Warrington PA 18976 (US)
(74) Representative: Forresters IP LLP
(86) International application number: PCT/US2017/012310
(87) International publication number: WO 2017/120319

(56) References cited:
- EP-A1- 0 474 599
- WO-A1-97/00353
- US-A1- 2011 277 249
- US-A1- 2012 029 561
- US-A1- 2014 025 106
- US-B2- 6 994 719

## Description

### FIELD OF THE INVENTION

The invention relates to a process for dyeing an article comprising an ultra-high weight polyethylene, with D&C Violet #2. A supercritical liquid is applied to the article with the dye at a specified temperature and pressure to yield the desired dyed article.

### BACKGROUND OF THE INVENTION

In a process known in the art, highly oriented ultra-high molecular weight polyethylene fibers are contacted with a dye bath at a temperature of 100-130°C for 20-60 minutes, with the dye bath consisting of an aqueous dispersion of a finely ground mixture of specific dyes and surfactants whereupon the moulded article is washed and dried. For good dyeing results the fibers are preferably modified by means of a plasma or corona treatment prior to the dyeing operation. An important drawback of the known process is that the dyed fibers, particularly if they are not first modified by means of a plasma or corona treatment, possess insufficient color intensity and that the color has insufficient resistance to rubbing and washing due to the dyes being located substantially at the surface of the fibers. US-A-2011/0277249 discloses the dyeing of UHMWPE fibers with D&C Violet #2 after chemical etching. The object of the invention is to provide a process that exhibits the aforementioned drawbacks to a lesser degree.

### SUMMARY OF THE INVENTION

Dyed articles, for example dyed sutures, and methods for dyeing articles are disclosed. The sutures may be made from ultra-high molecular weight polyethylene and dyed with D&C Violet #2.

The method for dyeing the articles includes contacting the article with a supercritical liquid and D&C Violet #2; applying a pressure from about 248.21 bar to about 262 bar (3,600 psi to about 3,800 psi); and applying a temperature from about 110°C to about 130°C for about 90 to about 180 minutes.

The resulting dyed article may have a color contrast of equal to or greater than about 90%. The color contrast is the color of the portion of the article that has been dyed on a scale between the color of the portion of the article when fully dyed (i.e., 100% contrast) to the pre-dyeing color of the portion of the article (i.e., 0% contrast).

### DETAILED DESCRIPTION

It has been found that very good dyeing results can be achieved by using the process of the invention. The dyes are brought into the article to a greater depth so that they cannot be removed by washing with water, and may only be removed with great difficulty by rubbing or even by boiling in hexane. A further advantage of the process of the invention is that the process is less laborious because no elaborate washing step or drying step is needed after dyeing. Yet another advantage is that the articles are colored more uniformly. It has specifically been found that good coloration occurs even in places that are difficult to access or inaccessible to dyes dispersed in water, such as those places where fibers are wound one over the other. This affords the highly attractive possibility of coloring fiber bobbins in their entirety.

Any known supercritical liquid may be used, such as carbon dioxide, dinitrogen oxide, ammonia, ethane or propane. Preferably, the supercritical liquid is carbon dioxide, because it becomes supercritical at a relatively low temperature and pressure and is harmless to man and the environment.

The choice of dye is of great importance. The dye must be soluble in the supercritical liquid, as well as exhibit a good affinity to highly oriented high-molecular-weight polyethylene articles. Good affinity means that the dye, in supercritical dyeing, is well able to penetrate into the article, resulting in a high color intensity and high resistance to rubbing and washing. The dye used herein is preferably D&C Violet #2, though other dyes may be used as well. D&C Violet #2 may also be used in combination with one or more additional dyes.

D&C Violet #2 has a violet hue and is in the anthraquinone dye chemical family. D&C Violet #2 is approved under 21 CFR §74.1602 and §74.2602. That is, it may be used safely for coloring externally applied drugs in amounts consistent with good manufacturing practices (21 CFR §74.1602) and color additive Ext. D&C Violet #2 may be safely used for coloring externally applied cosmetics in amounts consistent with good manufacturing practices (21 CFR §74.2602). Additional relevant technical data regarding D&C Violet #2 is presented in the following table:

**Table 1**

| | |
|---|---|
| *Hue:* **Violet** | |
| *Chemical Family:* **ANTHRAQUINONE DYE** | |
| CAS Number: 81-48-1 | |
| *Color Index Name:* **SOLVENT VIOLET 013** | |
| *Color Index Number:* **60725** | |
| *Appearance Odor:* **DARK PURPLE POWDER, ODORLESS** | |
| *Einecs Number:* **201-353-5** | |
| *Molecular Weight:* **329.35** | |
| *Pure Dye Content, (%):* **not less than 96%** | |
| *Light Fastness:* **4-5 (FAIR)** | |
| *REACH Compliance:* **05-2117992958-13-0000** | |
| *Arsenic:* **<3ppm** | |
| *Lead:* **<20ppm** | |
| *Mercury: <lppm* | |
| *Uses:* | *USE STATMENT:* Not to exceed by weight of suture: 0.2% in glycolic-lactic acid polyester synthetic absorbable sutures of 0.3% in polydioxanone synthetic absorbable sutures for use in general and ophthalmic surgery, and 0. 1% in poly(epsilon-caprolactone) absorbable sutures for use in general surgery. Externally applied Drugs and Cosmetics - consistent with Good Manufacturing Practice. |

| | |
|---|---|
| (Spectra Colors Corporation, D&C VIOLET 2 Technical Data Sheet and Material Safety Data Sheet, revised 21 September 2015, available at: www.SpectraColors.com) | |

D&C Violet #2 is identified as CAS 81-48-1 and EINECS 201-353-5, and has the chemical name 1-hydroxy-4-(p-toluidino)anthraquinone. It may also be known as CAS 4430-18-6, which is EINECS 224-618-7, having the chemical name sodium 4-[(9,10-dihydro-4-hydroxy-9,10-dioxo-1-anthryl)amino]toluene-3-sulphonate.

The dye may be mixed with the supercritical liquid and dissolved therein before contact with the article to be dyed, or the supercritical liquid and dye may be placed into contact with the article concurrently.

To that end, for the coloring of the article, the supercritical liquid is made to flow through a chamber or autoclave containing the article therein. The supercritical liquid flows through the article and then, after a designated time, leaves the treatment autoclave. Before the supercritical liquid is fed into the treatment autoclave, it may flow through a mass of pulverized dye in a saturator. Thereby, the liquid, which flows through the article, is loaded with the dye to a saturation point.

In the process according to the present invention, during the treatment period in which the liquid is continuously circulated, the treatment temperature, i.e., the temperature of the liquid flowing through the textile specimen may be set. During coloring according to the present invention, preferably, the supercritical liquid is heated before being loaded with the dyestuff. To this end, the supercritical liquid leaving the autoclave is first flown through the heat exchanger and, finally, through the saturator.

The dye that is used in accordance with the present invention must be soluble in supercritical liquid at the specified conditions of temperature and pressure. If the solubility of the dye is inadequate, it may be improved by adding moderators to the supercritical liquid. Exemplary moderators include but are not limited to toluene, methylpyrrolidone, Decalin, glycols or propanol.

However, a drawback of adding moderators is that they may be left behind on or in the polyethylene article on removing the supercritical liquid. This involves the risk of the moderators having an adverse effect on the properties of the article. A further drawback is that the moderators are often toxic, flammable and/or explosive. As a result, the important advantage of supercritical carbon dioxide that the solvent can be vented into the atmosphere without any safety precautions is lost. Surprisingly, it has been found that dyes having a solubility in acteone of at least 0.2 g/l at room temperature are well soluble in supercritical carbon dioxide without the use of moderators. In the most-preferred process, the supercritical solvent is carbon dioxide and the dye has a solubility in acetone of at least 0.2 g/l at room temperature.

A general description of a process for incorporating additives, including dyes, in polymeric fibers with the aid of a supercritical liquid is given in EP-B-0222207. It states that the polymeric fiber must assume a swollen condition in order to be able to incorporate the additives in the fiber, the degree of swelling of the fiber being at least 2 vol.% and preferably 5 vol.%. EP-A-474599 also discloses method for dyeing hydrophobic textile materials with disperse dyes in supercritical CO2.

In the process of the invention, the degree of swelling of the fibers during supercritical dyeing preferably is less than 1 vol.%. The degree of swelling is defined as the extent of the fiber's increase in volume on being contacted with a supercritical liquid. The advantage of this is that the strength of the fiber does not diminish unacceptably. A swelling degree of less than 1 vol.% is achieved by choosing a suitable supercritical liquid, for example CO₂, and a temperature from about 110°C to about 130°C. The process described in EP-B-0.222.207, in which the degree of swelling is greater than 2 vol.% and preferably greater than 5 vol.%, cannot be applied because at a degree of swelling of 2 or 5 vol.%, if such a degree of swelling could occur at all, the fibers almost completely lose their strength. Generally speaking, fibers having a crystallinity higher than 80% and a tensile strength greater than 2.5 GPa swell by less than 1 vol.% on being contacted with a supercritical liquid.

A article includes in particular fibers, monofilaments, multifilament yarns, staple fiber yarns, tapes, strips and films, and items made therefrom. The article preferably is a fiber or suture.

Part or all of an article, such as a suture made from ultra-high molecular weight polyethylene (i.e., extended chain, high-modulus or high-performance polyethylene) fiber and/or yarn (e.g., SPECTRA® Fiber and SPECTRA® Guard, from Honeywell International, Inc., Morris Township, NJ, or DYNEEMA® from Royal DSM N.V., Heerlen, the Netherlands) can be dyed according to the methods disclosed herein, or in, for example, PCT International Publication No. WO 97/00353 and U.S. Patent No. 5,953,780 except where modified as disclosed herein.

The article may be made from ultra-high molecular weight polyethylene (i.e., extended chain, high-modulus or high-performance polyethylene) fiber and/or yarn (e.g., SPECTRA® Fiber and SPECTRA® Guard, from Honeywell International, Inc., Morris Township, NJ, or DYNEEMA® from Royal DSM N.V., Heerlen, the Netherlands) and may also include, for example, single or multiple stainless steel alloys, nickel titanium alloys (e.g., Nitinol), cobalt-chrome alloys (e.g., ELGILOY® from Elgin Specialty Metals, Elgin, IL; CONICHROME® from Carpenter Metals Corp., Wyomissing, PA), nickel-cobalt alloys (e.g., MP35N® from Magellan Industrial Trading Company, Inc., Westport, CT), molybdenum alloys (e.g., molybdenum TZM alloy, for example as disclosed in International Pub. No. WO 03/082363 A2, published 9 October 2003, which is herein incorporated by reference in its entirety), tungsten-rhenium alloys, for example, as disclosed in International Pub. No. WO 03/082363, polymers such as polyethylene teraphathalate (PET), polyester (e.g., DACRON® from E. I. Du Pont de Nemours and Company, Wilmington, DE), poly ester amide (PEA), polypropylene, aromatic polyesters, such as liquid crystal polymers (e.g., Vectran, from Kuraray Co., Ltd., Tokyo, Japan), polytetrafluoroethylene (PTFE), expanded PTFE (ePTFE), polyether ketone (PEK), polyether ether ketone (PEEK), poly ether ketone ketone (PEKK) (also poly aryl ether ketone ketone), nylon, polyether-block co-polyamide polymers (e.g., PEBAX® from ATOFINA, Paris, France), aliphatic polyether polyurethanes (e.g., TECOFLEX® from Thermedics Polymer Products, Wilmington, MA), polyvinyl chloride (PVC), polyurethane, thermoplastic, fluorinated ethylene propylene (FEP), absorbable or resorbable polymers such as polyglycolic acid (PGA), poly-L-glycolic acid (PLGA), polylactic acid (PLA), poly-L-lactic acid (PLLA), polycaprolactone (PCL), polyethyl acrylate (PEA), polydioxanone (PDS), and pseudo-polyarnino tyrosine-based acids, extruded collagen, silicone, zinc, echogenic, radioactive, radiopaque materials, a biomaterial (e.g., cadaver tissue, collagen, allograft, autograft, xenograft, bone cement, morselized bone, osteogenic powder, beads of bone) any of the other materials listed herein or combinations thereof. Nonlimiting examples of useful radiopaque materials include barium sulfate, zinc oxide, titanium, stainless steel, nickel-titanium alloys, tantalum and gold.

The article before being dyed can be any color, for example, white.

Highly oriented means that the articles have been drawn so that the polymer chains run substantially completely parallel with the direction of drawing. It is preferred for the degree of orientation F to be at least 0.90, more preferably at least 0.95. The degree of orientation is defined by the formula F = (90° - H°/2)90°, where H° is the width at half the height of the scattering intensity along the Debye ring of the strongest reflection on the equator. Parts of highly oriented polyethylene have a high crystallinity of at least 70%, preferably of at least 80% and a tensile strength of at least 1.2 GPa and a tensile modulus of at least 40 GPa. Because of their high orientation and crystallinity, such parts are not dyeable or are poorly dyeable by the processes known to date. The present invention overcomes these deficiencies.

Ethylene homopolymer and copolymers of polyethylene and polypropylene are particularly suitable for use as polyethylene. The polyethylene used may also contain small amounts of one or more other polymers, particularly other alkene-1-polymers.

Preferably, the article is a high-molecular-weight linear polyethylene fiber having a tensile strength of at least 1.2 GPa and a tensile modulus of at least 40 GPa. In an embodiment, high-molecular weight means a molecular weight of at least 400,000 g/mol. An ultra-high weight polyethylene has a weight average molecular weight of at least 400 kg/mol.

Linear polyethylene here means polyethylene with fewer than 1 side chain per 100 carbon atoms, preferably fewer than 1 side chain per 300 carbon atoms. Furthermore, the polyethylene may contain up to 5 mol.% of one or more additional alkenes that may be copolymerized with it, such as propylene, butene, pentene, 4-methylpentene, and octene.

Preferably, use is made of polyethylene fibers consisting of polyethylene filaments that have been prepared by a gel spinning process as described in for instance GB-A-2042414 and GB-A-2051667. Basically, this process comprises preparing a solution of a polyethylene having a high intrinsic viscosity, spinning the solution to filaments at a temperature above the dissolution temperature, cooling the filaments below the gelating temperature so that gelation takes place and drawing the filaments before, during or after removal of the solvent.

The preparation of a highly oriented polyethylene article usually includes two or more drawing steps, often at progressively higher temperature and lower drawing rates.

In another embodiment of the process of the invention the article is redrawn after dyeing. Surprisingly, it has been found that the color intensity is enhanced by the drawing itself. A good enhancement of the color intensity can in principle be achieved in a drawing operation that does not or does not substantially contribute to the strength or modulus, for example at most 20 or 10%. However, from a process engineering point of view it is advantageous to carry out the drawing operation such that the strength and modulus are also further improved. The manner in which this can be achieved is known to one skilled in the art. The presence of dyes has no adverse effect on the tensile strength and tensile modulus attainable by drawing. A further advantage of this embodiment even is that the dyed articles obtained have a higher tensile strength and tensile modulus than articles that are drawn under comparable conditions and are dyed afterwards.

In a preferred embodiment of the above process a solvent for polyethylene is applied to the article prior to or during drawing. The advantage of this is that the color intensity increases further. Preferably, this solvent is a nonvolatile substance such as paraffin oil. The advantage of this is that the solvent concentration remains virtually constant during drawing.

A good color intensity can be obtained by drawing the dyed article, whether or not in the presence of a solvent, even if the article is ultrahighly oriented prior to drawing and/or if dyes are used that give too weak a color intensity without these measures. Ultrahighly oriented article here and hereafter means an article having a tensile strength of at least 2.5 GPa, preferably at least 3 GPa and more preferably 3.5 GPa.

In another embodiment of the process, the article is a polyethylene fiber whose tensile strength prior to drawing is between 0.7 GPa and 2.5 GPa. More preferably, the tensile strength then is between 0.7 and 2 GPa, more preferably between 0.7 and 1.5 GPa. It has been found that, with this measure, a still higher color intensity can be obtained. In this case, the article is preferably drawn after dyeing to a strength of at least 2.5 GPa, preferably at least 3 GPa, still more preferably 3.5 GPa.

The tensile strength of the article preferably is at least 0.7 GPa, because the article needs to have a certain minimum strength so that it can be used under high pressure and temperature conditions.

The drawing of the fiber may take place in a wide temperature range. At low temperatures, however, the drawing rate should be low in order to prevent premature breakage. If a higher drawing rate is to be obtained, the drawing temperature is preferably chosen within 10°C below the melting point of the article and the drawing rate is below 1 sec⁻¹. For dyes with a low molecular weight, lower than, for example, 300 g/mol or more particularly lower than 250 g/mol, the post-drawing temperature is preferably chosen so low that sublimation or sweating of the dye out of the fiber remains limited.

Surprisingly, it has been found that highly oriented polyethylene articles, especially fibers, obtain a higher color intensity if they undergo a mechanical operation prior to dyeing. Mechanical operation particularly means exposing the fibers to a flexural load in such a way that a component force acts on the fiber perpendicularly to the direction of the fiber. This situation arises when for instance a taut fiber is pulled through an eye at a certain bending angle. In an still better embodiment of the process of the invention the article is therefore exposed to a flexural load prior to being dyed.

Since, almost without exception, a flexural load also occurs in the processing of a fiber to an article by, for instance, taslanization, crimping, twisting, doubling, weaving, knitting or braiding, the aforementioned advantages are also obtained when the polyolefinic articles are dyed after they have been incorporated into an article.

The invention therefore relates to a process for dyeing articles containing a highly oriented article substantially consisting of a polyethylene having a weight average molecular weight of at least 400 kg/mol, characterized in that the article is contacted, at a temperature of between 100 and 130°C, maintaining said temperature for about 90 to about 180 minutes with a supercritical liquid in which D&C Violet #2 is dissolved. Some examples of such articles are blended yarns, combined yarns, knitted fabrics, woven fabrics, felts, ropes, fishing lines, sails, gloves and helmets.

The aforementioned preferred embodiments for the dyeing of a polyethylene article are analogously applicable to the dyeing of articles containing such articles.

In an embodiment of the aforementioned process that is particularly preferred the article, more specifically a rope or a fishing line, substantially consists of partially drawn polyethylene fibers having a tensile strength of at least 0.7 GPa and a tensile modulus of at least 7 GPa and the article is post-drawn after dyeing. Further advantages of the latter embodiment are that loss in strength caused by assembly of the article from the polyethylene fibers by for instance twisting, plying or braiding is offset in the post-drawing step so that the article has both a higher color intensity and a much higher strength and dimensional stability.

The temperature in the process of the invention is about 110°C to about 130°C. Below 100°C, the solubility of the dyes in the supercritical liquid, especially in carbon dioxide, is too low. Above 130°C, excessive shrinkage and strength loss occurs in the polyethylene parts. In order to alleviate strength losses it is preferred for the articles to be dyed as they are taut. A suitable method of dyeing taut fibers is to tightly wind a number of layers of the fibers onto a bobbin and then to dye them.

The pressure is chosen in dependence on the liquid used. For supercritical carbon dioxide, the pressure preferably is at least about 20 MPa (about 2,900 psi), more preferably at least about 25 MPa (about 3,626 psi). The pressure may be from about 24.82 MPa to about 26.20 MPa (3,600 psi to about 3800 psi). The solubility of the dyes is higher at such high pressures. After dyeing, the pressure preferably is reduced at a rate of at most 1.5 MPa per minute, more preferably at most 1 MPa per minute. If the pressure is reduced discontinuously, the pressure is reduced in each step preferably by not more than 1.5 MPa, preferably not more than 1 MPa, with the intervals between each step being long enough not to exceed the said highest rate of pressure reduction. It has been found that too rapid pressure reduction may lead to damage to the article. Too rapid expansion may lead to significant strength loss especially in partially drawn polyethylene fibers with a low tensile strength (between 0.7 and 2 GPa).

The dyeing time is desirably chosen so that the color intensity is as high as possible. The article is processed with the dye at the specified time and pressure for about 90 minutes to about 180 minutes.

In an embodiment, after contacting the article with the supercritical liquid and the dye, the mixture may be exposed to about 24.82 MPa to about 26.20 MPa (3,600 psi to about 3800 psi). of pressure; and from about 110°C to about 130°C; from about 90 to about 180 minutes. The pressure may be applied concurrent with, overlapping, or preceding the application of the temperature.

The amount of dye weighed into the article generally amounts to from about 0.1 to about 5 wt.% relative to the weight of the article.

Preferably, a somewhat larger amount of dye is added than the amount which is soluble in the supercritical liquid. Preferably, the solution is stirred in order to obtain good, homogeneous coloration.

In an embodiment, with the used supercritical fluid, in this example, carbon dioxide CO₂, with an increased temperature a greater amount of dye can be dissolve. As a result of the liquid circulation, with the supercritical liquid being first heated and then loaded with the dye, the article is impinged with a supercritical liquid having a constantly increasing dye concentration. The increasing concentration difference between the dye-absorbing article and the dye-applying liquid favorably influences the dye diffusion process. The treatment duration is shorter in comparison with isothermal dyes.

Besides one or more dyes, other substances may optionally be added to the supercritical solvent; these include UV stabilizers for improving the light-fastness of the dyes or crosslinking reagents for improving the creep resistance and the oxystability of the polyethylene articles.

After processing, the dyed article may have a color contrast of equal to or greater than about 90%. The color contrast is the color of the portion of the article that has been dyed on a scale between the color of the portion of the article when fully dyed (i.e., 100% contrast) to the pre-dyeing color of the portion of the article (i.e., 0% contrast).

The article may be completely covered, striped, dotted, or combinations thereof by the dye. For example, the article may have one or more helical stripes of dye.

The article may be attached to other elements, such as needles, hooks, anchors, or combinations thereof at one or both terminal ends of each article. The article may also be used with any medical device known in the art. The device may be made from substantially 100% polyether ether ketone (PEEK), substantially 100% titanium or titanium alloy, or combinations thereof. Any or all elements of the article and/or other devices or apparatuses described herein, may have and/or be completely or partially coated with agents for cell ingrowth.

The elements of the article can be filled, coated, layered and/or otherwise made with and/or from cements, fillers, and/or glues known to one having ordinary skill in the art and/or a therapeutic and/or diagnostic agent. Any of these cements and/or fillers and/or glues may be osteogenic and osteoinductive growth factors.

Examples of such cements and/or fillers includes bone chips, demineralized bone matrix (DBM), calcium sulfate, coralline hydroxyapatite, biocoral, tricalcium phosphate, calcium phosphate, polymethyl methacrylate (PMMA), biodegradable ceramics, bioactive glasses, hyaluronic acid, lactoferrin bone morphogenic proteins (BMPs) such as recombinant human bone morphogenetic proteins (rhBMPs), other materials described herein, or combinations thereof.

The agents within these matrices, i.e., cements, fillers, and/or glues, can include any agent disclosed herein or combinations thereof, including radioactive materials; radiopaque materials; cytogenic agents; cytotoxic agents; cytostatic agents; thrombogenic agents, for example polyurethane, cellulose acetate polymer mixed with bismuth trioxide, and ethylene vinyl alcohol; lubricious, hydrophilic materials; phosphor cholene; anti-inflammatory agents, for example non-steroidal anti-inflammatories (NSAIDs) such as cyclooxygenase-1 (COX-1) inhibitors (e.g., acetylsalicylic acid, for example ASPIRIN® from Bayer AG, Leverkusen, Germany; ibuprofen, for example ADVIL® from Wyeth, Collegeville, PA; indomethacin; mefenamic acid), COX-2 inhibitors (e.g., VIOXX® from Merck & Co., Inc., Whitehouse Station, NJ; CELEBREX® from Pharmacia Corp., Peapack, NJ; COX-1 inhibitors); immunosuppressive agents, for example Sirolimus (RAPAMUNE®, from Wyeth, Collegeville, PA), or matrix metalloproteinase (MMP) inhibitors (e.g., tetracycline and tetracycline derivatives) that act early within the pathways of an inflammatory response. Examples of other agents are provided in Walton et al, Inhibition of Prostoglandin E2 Synthesis in Abdominal Aortic Aneurysms, Circulation, July 6, 1999, 48-54; Tambiah et al, Provocation of Experimental Aortic Inflammation Mediators and Chlamydia Pneumoniae, Brit. J. Surgery 88 (7), 935-940; Franklin et al, Uptake of Tetracycline by Aortic Aneurysm Wall and Its Effect on Inflammation and Proteolysis, Brit J. Surgery 86 (6), 771-775; Xu et al, Sp 1 Increases Expression of Cyclooxygenase-2 in Hypoxic Vascular Endothelium, J.Biological Chemistry 275 (32) 24583-24589; and Pyo et al, Targeted Gene Disruption of Matrix Metalloproteinase-9 (Gelatinase 13) Suppresses Development of Experimental Abdominal Aortic Aneurysms, J. Clinical Investigation 105 (11), 1641-1649

In an embodiment, the apparatus for dying an article disclosed herein includes:
a. at least one autoclave for receiving an article,
b. at least one saturator for loading the fluid with a dye,
c. at least one separator,
d. a heat exchanger with associated heating and cooling device,
e. a condensator with an associate cooling device and a collector arranged downstream of the condensator, and
f. a pump.

In the apparatus, the above mentioned devices may be connected with conduits and armature that the fluid, on one hand, can circulate through the dye autoclave, the pump, the heat exchanger, the saturator or, on the other hand, through the dye autoclave, the relief valve, the condensator, the collector, and the pump.

The autoclave itself may include means which provides for the fluid flow through the yarn spool and which provides for flow of fluid. If necessary, the autoclave can be so formed that it can receive yarn spool columns and/or a flat product wound on a roller.

The dye autoclave may be equipped with a mechanized cover lock. For the autoclaves with an inner diameter of 1 meter, commercial quick-acting clamps for high pressures from 300 bar to 700 bar are available. For larger diameters mechanized segmented annular locks are used. Those are suitable for all treatment pressures which were here considered.

Supercritical fluids may be fed with piston and rotary pumps, which are specifically designed for this use. In the process described above, rotary pumps are used when, e.g., a plurality of long spool columns are treated simultaneously.

## Claims

1. A method of dyeing an article comprising an ultra-high molecular weight polyethylene (UHMWPE), comprising:
contacting the article with a supercritical liquid and D&C Violet #2;
applying a temperature from about 110°C to about 130°C; and
maintaining said temperature for about 90 to about 180 minutes.

2. The method of claim 1, further comprising applying a pressure from about 248.21 to about 262 bar (about 3600psi to about 3800 psi)

3. The method of claim 1, wherein the dyed article has a color contrast of at least about 90%.

4. The method of any one of claims 1 through 3, wherein the article is a suture.

5. The method of claim 1, wherein D&C Violet #2 is weighed into the article in an amount about 0.1 to about 5 wt.% relative to the weight of the article.

6. The method of claim 2, further comprising, after dyeing, reducing the pressure at a rate of equal to or less than 1.5 MPa per minute.

## Patentansprüche

1. Verfahren zur Färbung eines Artikels umfassend ein Polyethylen mit ultrahohem Molekulargewicht (UHMWPE), umfassend:
Kontaktieren des Artikels mit einer überkritischen Flüssigkeit und D&C Violet #2;
Anwenden einer Temperatur von etwa 110 °C bis etwa 130 °C; und
Aufrechterhalten besagter Temperatur für etwa 90 bis etwa 180 Minuten.

2. Verfahren nach Anspruch 1, ferner umfassend Anwenden eines Drucks von etwa 248,21 bis etwa 262 bar (etwa 3600 psi bis etwa 3800 psi).

3. Verfahren nach Anspruch 1, wobei der gefärbte Artikel einen Farbkontrast von mindestens etwa 90 % aufweist.

4. Verfahren nach einem der Ansprüche 1 bis 3, wobei der Artikel eine Naht ist.

5. Verfahren nach Anspruch 1, wobei D&C Violet #2 in einer Menge von 0,1 bis etwa 5 Gew.-% relativ zum Gewicht des Artikels in den Artikel eingewogen wird.

6. Verfahren nach Anspruch 2, ferner umfassend, nach der Färbung, Reduzieren des Drucks mit einer Rate von gleich oder weniger als 1,5 MPa pro Minute.

## Revendications

1. Procédé de teinture d'un article comprenant un polyéthylène de poids moléculaire ultra élevé (UHMWPE), comprenant :
la mise en contact de l'article avec un liquide supercritique et le D&C Violet #2 ;
l'application d'une température d'environ 110 °C à environ 130 °C ; et
le maintien de ladite température pendant environ 90 à 180 minutes.

2. Procédé selon la revendication 1, comprenant en outre l'application d'une pression d'environ 248,21 à environ 262 bars (environ 3600 psi à environ 3800 psi)

3. Procédé selon la revendication 1, dans lequel l'article teint présente un contraste de couleur d'au moins environ 90 %.

4. Procédé selon l'une quelconque des revendications 1 à 3, dans lequel l'article est une suture.

5. Procédé selon la revendication 1, dans lequel le violet D&C No 2 est pesé dans l'article en une proportion d'environ 0,1 à environ 5 % en poids par rapport au poids de l'article.

6. Procédé selon la revendication 2, comprenant en outre, après la teinture, la réduction de la pression à un taux égal ou inférieur à 1,5 MPa par minute.
